# EUROPEAN PATENT APPLICATION

(11) **EP 4 725 515 A1**
(43) Date of publication of application: **15.04.2026**
(21) Application number: 24823375.1
(22) Date of filing: 11.06.2024
(51) Int. Cl.: A61L 33/02, A61L 29/02, A61L 29/04, A61L 31/02, A61L 31/04, A61L 33/06

(54) **MEDICAL INSTRUMENT AND PRODUCTION METHOD FOR SAME**

(30) Priority: 15.06.2023 JP 2023098416
(71) Applicant: Terumo Kabushiki Kaisha, Tokyo 151-0072 (JP)
(72) Inventor: LIU, Yihua, Ashigarakami-gun, Kanagawa 259-0151 (JP); KITAHARA, Yoko, Ashigarakami-gun, Kanagawa 259-0151 (JP); HIRAKI, Akihiro, Ashigarakami-gun, Kanagawa 259-0151 (JP)
(74) Representative: Prüfer & Partner mbB Patentanwälte · Rechtsanwälte
(86) International application number: PCT/JP2024/021189
(87) International publication number: WO 2024/257760

(57) **Abstract**

An object of the present invention is to provide means capable of imparting excellent functionality (for example, antithrombogenicity) in a medical device (particularly, a medical device including a metal base material). The object is achieved with a medical device including: a metal base material; and a functional layer having a polymer including a constituent unit A derived from a bonding monomer having a phosphoric acid group or a phosphonic acid group and an ethylenically unsaturated group, which is bonded to the base material, and a functional polymer chain formed of a constituent unit B derived from a functional monomer, with a terminal of the functional polymer chain being bonded to the constituent unit A.

## Description

### Technical Field

The present invention relates to a medical device and a method for manufacturing the medical device.

### Background Art

In recent years, medical materials using various metal materials have been studied, and are expected to be used for stent grafts, covered stents, stents, guide wires, cardiac pacemakers, and the like. In this regard, a metal material that is foreign matter for a living body is used in contact with body tissues and body fluids such as blood. Therefore, the medical materials are required to have biocompatibility. The biocompatibility required for medical materials varies depending on the purpose and use method, and the medical materials used as materials in contact with blood are required to have characteristics (antithrombogenicity), such as suppressing a blood coagulation system, suppressing the adhesion and activation of platelets, and suppressing the activation of a complement system.

In the related art, a method for covering (coating) a base material constituting a medical device with an antithrombotic material has been mainly adopted as a method for imparting antithrombogenicity to the medical device. For example, WO 2020/166605 A (corresponding to US 2022/0135806 A) discloses a copolymer-containing varnish containing a copolymer of a phosphoric acid-containing compound having a phosphoric acid group and a (meth)acryloyl group and a compound having a cationic monovalent organic group and a (meth)acryloyl group. According to the above-described literature, a copolymer is synthesized using a phosphoric acid-containing compound having a purity of 70 mass% or more, and gelation of the copolymer in the copolymer-containing varnish is thus suppressed. Furthermore, the above-described literature also describes that a coating film having biocompatibility can be formed by applying the copolymer-containing varnish to a surface of a base material and drying the coating film.

### Summary of Invention

### Technical Problem

However, according to the studies conducted by the inventors, it has been found that the copolymer described in the above-described literature is likely to aggregate, and a copolymer-containing varnish having sufficient handleability may not be obtained. Since medical devices manufactured using a copolymer-containing varnish having low handleability due to aggregation of a copolymer may have insufficient functionality (for example, antithrombogenicity), it is desired to provide a new method for imparting functionality (for example, antithrombogenicity) to the medical devices.

Therefore, an object of the present invention is to provide means capable of imparting excellent functionality (for example, antithrombogenicity) in a medical device (particularly, a medical device including a metal base material).

### Solution to Problem

The inventors have conducted intensive studies to solve the above problems. As a result, the inventors have found that the above problems can be solved by immobilizing a functional polymer chain on a surface of a metal base material via a predetermined constituent unit, and have completed the present invention.

That is, the object can be achieved by the present invention having the following configuration, and the present invention includes the following aspects and forms.

According to one aspect of the present invention,
1. there is provided a medical device including:
   a metal base material; and
   a functional layer having a polymer including a constituent unit A derived from a bonding monomer having a phosphoric acid group or a phosphonic acid group and an ethylenically unsaturated group, which is bonded to the base material, and a functional polymer chain formed of a constituent unit B derived from a functional monomer, with a terminal of the functional polymer chain being bonded to the constituent unit A.
2. In the medical device according to 1., a ratio of a number of the constituent units B to a number of the constituent units A in the polymer is preferably 30 or more.
3. In the medical device according to 1. or 2., the bonding monomer is preferably one or more kinds selected from monomers represented by Formulae (I) and (II): 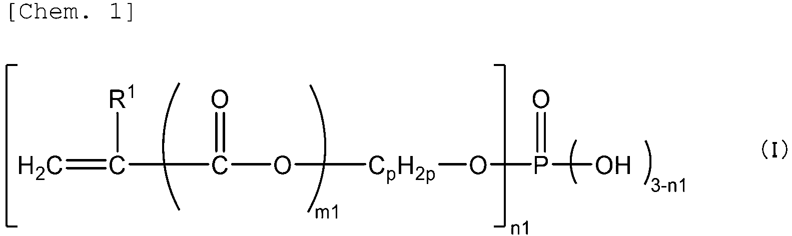 In Formula (I),
   R¹ represents a hydrogen atom or a methyl group,
   p represents an integer of 0 to 20,
   m1 represents 0 or 1, and
   n1 represents 1 or 2; and 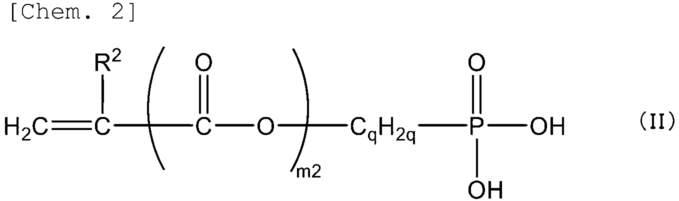
   In Formula (II),
   R² represents a hydrogen atom or a methyl group,
   q represents an integer of 0 to 20, and
   m2 represents 0 or 1.
4. In the medical device according to any one of 1. to 3., the bonding monomer is preferably one or more kinds selected from 2-(meth)acryloyloxyethyl acid phosphate, bis[2-((meth)acryloyloxy)ethyl] phosphate, 11-phosphonoundecyl (meth)acrylate, vinylphosphonic acid, and allylphosphonic acid.
5. In the medical device according to any one of 1. to 4., the functional monomer is preferably one or more kinds selected from monomers represented by Formulae (III) to (V) : 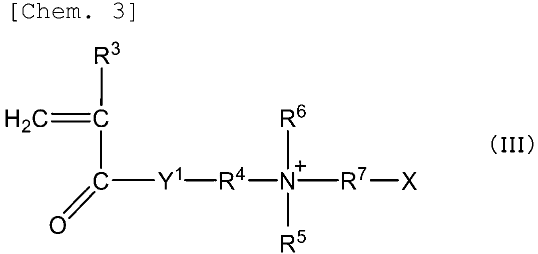 In Formula (III),
   R³ represents a hydrogen atom or a methyl group,
   R⁴ represents a linear or branched alkylene group having 1 to 6 carbon atoms,
   R⁵ and R⁶ each independently represent a linear or branched alkyl group having 1 to 4 carbon atoms,
   R⁷ represents a linear or branched alkylene group having 1 to 4 carbon atoms,
   X represents -COO⁻ or -SO₃⁻, and
   Y¹ represents an oxygen atom or -NH-; 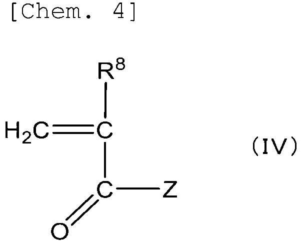
   In Formula (IV),
   R⁸ represents a hydrogen atom or a methyl group,
   Z represents a group represented by Formula: -O-(R⁹O)ₘ-R¹⁰, a group represented by Formula: -O-(CH₂CHOH)ₙ-CH₂OH, or a tetrahydrofurfuryloxy group, in which R⁹ each independently represents a linear or branched alkylene group having 1 to 4 carbon atoms, R¹⁰ represents a linear or branched alkyl group having 1 to 4 carbon atoms, m represents an integer of 1 to 5, and n represents an integer of 1 to 5; and 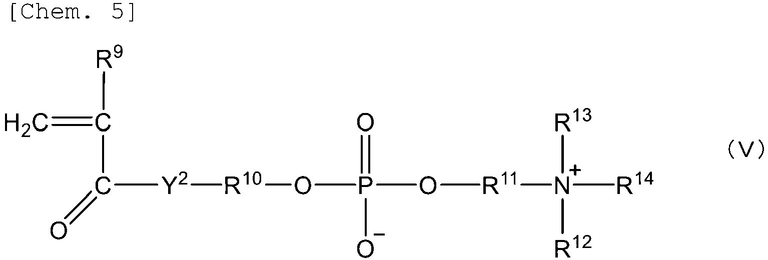
   In Formula (V),
   R⁹ represents a hydrogen atom or a methyl group,
   R¹⁰ and R¹¹ each independently represent a linear or branched alkylene group having 1 to 6 carbon atoms,
   R¹² to R¹⁴ each independently represent a linear or branched alkyl group having 1 to 4 carbon atoms, and
   Y² represents an oxygen atom or -NH-.
6. In the medical device according to any one of 1. to 5., the functional monomer is preferably one or more kinds selected from N-(meth)acryloyloxymethyl-N,N-dimethylammonium-α-N-methylcarboxybetaine, N-(meth)acryloyloxyethyl-N,N-dimethylammonium-α-N-methylcarboxybetaine, N-(meth)acryloyloxypropyl-N,N-dimethylammonium-α-N-methylcarboxybetaine, N-(meth)acryloyloxymethyl-N,N-diethylammonium-α-N-methylcarboxybetaine, N-(meth)acryloyloxyethyl-N,N-diethylammonium-α-N-methylcarboxybetaine, N-(meth)acryloyloxypropyl-N,N-diethylammonium-α-N-methylcarboxybetaine, methoxymethyl acrylate, 2-methoxyethyl acrylate, 3-methoxypropyl acrylate, ethoxymethyl acrylate, ethoxyethyl acrylate, 2-(2-ethoxyethoxy)ethyl acrylate, ethoxypropyl acrylate, ethoxybutyl acrylate, propoxymethyl acrylate, butoxyethyl acrylate, methoxybutyl acrylate, tetrahydrofurfuryl acrylate, methoxymethyl methacrylate, methoxyethyl methacrylate, ethoxymethyl methacrylate, ethoxyethyl methacrylate, 2-(2-ethoxyethoxy)ethyl methacrylate, propoxymethyl methacrylate, butoxyethyl methacrylate, tetrahydrofurfuryl methacrylate, glycerol (meth)acrylate, and 2-methacryloyloxyethyl phosphorylcholine.
7. In the medical device according to any one of 1. to 6., the metal is preferably one or more kinds selected from the group consisting of gold, platinum, silver, copper, nickel, cobalt, titanium, iron, aluminum, tin, stainless steel, nickel-titanium alloys, nickel-cobalt alloys, cobalt-chromium alloys, and zinc-tungsten alloys.
8. In the medical device according to any one of 1. to 7., the medical device is preferably a stent graft, a covered stent, a stent, a guide wire, or a cardiac pacemaker.

According to another aspect of the present invention,
9. there is provided a method for manufacturing the medical device according to any one of 1. to 8., including:
irradiating at least a part of a surface of the base material with plasma;
bringing the bonding monomer into contact with the base material after irradiation with the plasma; and
bringing the functional monomer into contact with the base material after contact with the bonding monomer to form the functional polymer chain on the surface of the base material via the constituent unit A derived from the bonding monomer.

### Brief Description of Drawings

Fig. 1 is a view for explaining immobilization of an antithrombotic material in the present invention.
Fig. 2 is a partial cross-sectional view schematically showing a structure according to a representative embodiment of a medical device according to the present invention.
Fig. 3 is a partial cross-sectional view schematically showing a different configuration example of the structure as an application example of the embodiment of Fig. 2.
Fig. 4 is an SEM image of a NiTi base material (NiTi (not plasma-treated)) before a plasma treatment, observed by a scanning electron microscope.
Fig. 5 is an SEM image of a NiTi base material (NiTi-PHA-CBA) in which CBA is polymerized via PHA, observed by a scanning electron microscope.
Fig. 6 is an SEM image of a NiTi base material (NiTi-PHA-MEA) in which MEA is polymerized via PHA, observed by a scanning electron microscope.
Fig. 7 is an SEM image of a NiTi base material (NiTi-PHA-MC3A) in which MC3A is polymerized via PHA, observed by a scanning electron microscope.
Fig. 8 is an SEM image of a NiTi base material (NiTi-PHA-MPC) in which MPC is polymerized via PHA, observed by a scanning electron microscope.

### Description of Embodiments

According to one embodiment of the present invention, a medical device including a metal base material and a functional layer is provided. The functional layer has a polymer including a constituent unit A derived from a bonding monomer having a phosphoric acid group or a phosphonic acid group and an ethylenically unsaturated group, which is bonded to the base material, and a functional polymer chain formed of a constituent unit B derived from a functional monomer, with a terminal of the functional polymer chain being bonded to the constituent unit A. According to the present embodiment, excellent functionality (for example, antithrombogenicity) can be imparted to the medical device including the metal base material.

Hereinafter, a medical device having an antithrombotic layer as a functional layer will be described in detail as an example (in this case, the constituent unit B is derived from an antithrombotic monomer). However, the present invention can be applied to a medical device having any functional layer such as an antithrombotic layer, an antibacterial layer, an anti-inflammatory layer, or a biodegradable layer.

Surprisingly, the inventors have found that a medical device having such a configuration has excellent antithrombogenicity (that is, it is possible to effectively suppress the adhesion of proteins, cells, and the like that can constitute clots). Here, the mechanism of exerting the above-described actions and effects with the configuration according to the present invention is presumed as follows.

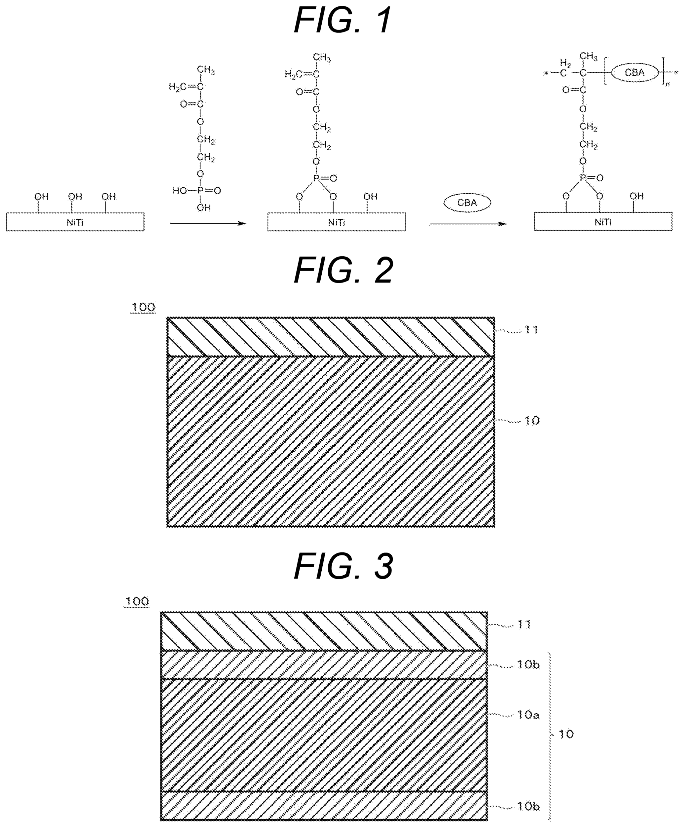

Fig. 1 is a view for explaining immobilization of an antithrombotic material in the present invention. As shown in Fig. 1, in the medical device according to the present invention, a plurality of constituent units B derived from an antithrombotic monomer (for example, N-methacryloyloxyethyl-N,N-dimethylammonium-α-N-methylcarboxybetaine (CBA)) are immobilized on a surface of a metal base material (for example, NiTi base material) via a constituent unit A derived from a bonding monomer (for example, 2-methacryloyloxyethyl acid phosphate (PHA)) having a phosphoric acid group or a phosphonic acid group and an ethylenically unsaturated group. According to the present invention, using a bonding monomer having a phosphoric acid group or a phosphonic acid group, an ethylenically unsaturated group can be constructed on a surface of a metal base material via covalent bonding. In addition, an antithrombotic layer can be formed on the surface of the metal base material by polymerizing an antithrombotic monomer with the ethylenically unsaturated group. Since the antithrombotic layer is bonded to the metal base material via covalent bonding, the antithrombogenicity can be maintained for a longer period of time compared to a method for immobilizing an antithrombotic material by covering (coating) according to the related art. Therefore, a medical device having excellent durability can be provided. In addition, in the method for immobilizing an antithrombotic material by covering (coating) according to the related art, a copolymer is previously synthesized, and then applied to the surface of the base material. However, according to the present invention, there is no need to previously synthesize a copolymer, and thus a problem such as a decrease in handleability due to aggregation does not occur in principle. Therefore, a uniform antithrombotic layer can be formed on the surface of the metal base material, and a medical device having excellent antithrombogenicity can be provided. Furthermore, there is also an advantage that the functional layer can be formed using not only antithrombotic monomers but also various functional monomers.

Note that the above-described mechanism is an inference and does not limit the technical scope of the present invention at all.

In the present specification, the medical device having the above-described configuration is also simply referred to as the "medical device according to the present invention" or the "medical device". In addition, in the present specification, the "constituent unit A derived from a bonding monomer having a phosphoric acid group or a phosphonic acid group and an ethylenically unsaturated group" is also simply referred to as the "constituent unit A according to the present invention" or the "constituent unit A". In the present specification, the "constituent unit B derived from a functional monomer" is also simply referred to as the "constituent unit B according to the present invention" or the "constituent unit B". In the present specification, the polymer including the constituent unit A and the constituent unit B is also simply referred to as the "polymer according to the present invention" or the "polymer".

In the present specification, in a case in which a constituent unit is determined as being "derived" from a certain monomer, it means that the constituent unit is a constituent unit generated by a condensation reaction of a reactive group of a corresponding monomer and/or generated by cleavage of one bond of an ethylenically unsaturated group (polymerizable unsaturated double bond) of the corresponding monomer.

In the present specification, the term "(meth)acryl" refers to both acryl and methacryl. Therefore, for example, the term "(meth)acrylic acid" refers to both acrylic acid and methacrylic acid. Similarly, the term "(meth)acryloyl" refers to both acryloyl and methacryloyl. Therefore, for example, the term "(meth)acryloyl group" refers to both acryloyl group and methacryloyl group. Further similarly, the term "(meth)acrylate" refers to both acrylate and methacrylate. Therefore, for example, the term "alkoxyalkyl (meth)acrylate" refers to both alkoxyalkyl acrylate and alkoxyalkyl methacrylate.

In the present specification, a range of "X to Y" includes X and Y, and indicates "X or more and Y or less". Also, "A and/or B" indicates including A, B, and combinations thereof. Unless otherwise specified, operations and measurements of physical properties and the like are performed at room temperature (20 to 25°C) with a relative humidity of 40 to 50% RH.

Hereinafter, embodiments of the present invention will be described. Note that the present invention is not limited only to the following embodiments, and various modifications can be made within the scope of claims. In addition, the embodiments described in the present specification can be optionally combined with each other to form another embodiment. The dimensional ratios in the drawings are exaggerated for convenience of description and may be different from actual ratios. In addition, in a case in which the embodiments of the present invention are described with reference to the drawings, the same elements are denoted by the same reference numerals in the description of the drawings, and redundant description will be omitted.

### [Medical Device]

Hereinafter, a configuration of the medical device according to one embodiment of the present invention will be described with reference to the accompanying drawings.

Fig. 2 is a partial cross-sectional view schematically showing a structure according to a representative embodiment of the medical device according to the present invention. Fig. 3 is a partial cross-sectional view schematically showing a different configuration example of the structure as an application example of the present embodiment.

As shown in Figs. 2 and 3, a medical device 100 according to one embodiment of the present invention includes a metal base material 10 and an antithrombotic layer 11 formed on at least a part of the metal base material 10. Here, in the medical device 100 according to the present embodiment, the metal base material (also simply referred to as the "base material") 10 and the antithrombotic layer 11 are directly laminated in this order. That is, the base material 10 and the antithrombotic layer 11 are laminated adjacent to each other in this order.

In the present embodiment, the polymer (particularly, the phosphoric acid group or the phosphonic acid group of the constituent unit A derived from the bonding monomer) constituting the antithrombotic layer and a surface (particularly, the hydroxyl group (-OH) generated by a plasma treatment) of the metal base material are chemically bonded to each other by covalent bonding, and thus it is possible to maintain antithrombogenicity for a long period of time.

In addition, in the present specification, the reason for the phrase "the antithrombotic layer is formed on at least a part of the metal base material" is that, in a stent graft, a covered stent, a stent, a guide wire, a cardiac pacemaker, or the like, which are examples of applications of the medical device, it is not always necessary to impart antithrombogenicity to all of the surfaces (entire surfaces) of the medical device, and the antithrombotic layer only needs to be formed on a surface portion (sometimes a part of the surface, sometimes the entire surface) required to have antithrombogenicity. Therefore, the antithrombotic layer is preferably formed on at least a portion of the base material 10 that is in contact with blood. For example, in a case in which the medical device is a stent graft, a covered stent, a stent, or a cardiac pacemaker, the antithrombotic layer is preferably formed on the entire surface of the base material.

### <<Metal Base Material (Metal Base Material Layer)>>

The base material 10 constituting the medical device according to one embodiment of the present invention is a metal base material. As the metal base material, an optimal base material can be appropriately selected according to characteristics (for example, mechanical strength and the like) required for a medical device such as a stent graft, a covered stent, a stent, a guide wire, or a cardiac pacemaker. As an example, those having characteristics required for the base material such as a mechanical strength, elasticity, and toughness are preferable.

At least the surface of the base material 10 may be made of a metal material. As an example, as shown in Fig. 2, the entire base material may be made (formed) of a metal material. In addition, as shown in Fig. 3, a structure may be provided in which a base material surface layer 10b made of a metal material is constituted (formed) on a surface of a base material core portion 10a made of a material such as plastic (polymer material) or a ceramic material. In addition, the base material 10 may be formed by combining the base material core portion 10a and the base material surface layer 10b (appropriate reaction treatment). Therefore, the base material core portion 10a may be a multilayer structure obtained by laminating different materials in multiple layers, a structure (combined body) in which members made of different materials for portions of the medical device are joined together, or the like. Another middle layer (not shown) may be further formed between the base material core portion 10a and the base material surface layer 10b. Furthermore, the base material surface layer 10b may also be a multilayer structure obtained by laminating different materials in multiple layers, a structure (combined body) in which members made of different materials for portions of the medical device are joined together, or the like, as long as the surface of the base material surface layer 10b is made (formed) of a metal material.

The metal material constituting (forming) the base material 10 and the base material surface layer 10b is not particularly limited, and metal materials generally used for medical devices such as stent grafts, covered stents, stents, guide wires, and cardiac pacemakers are used. Specific examples thereof include gold, platinum, silver, copper, nickel, cobalt, titanium, iron, aluminum, tin, stainless steel (for example, SUS304, SUS314, SUS316, SUS316L, SUS420J2, SUS630, and SUS631), nickel-titanium alloys, nickel-cobalt alloys, cobalt-chromium alloys, and zinc-tungsten alloys. These may be used singly or in combination of two or more kinds thereof. The metal material can be appropriately selected according to the application of the medical device. As the metal material constituting (forming) a base material of a stent graft, a covered stent, a stent, a guide wire, or a cardiac pacemaker that is an example of the use applications, one or more kinds selected from the group consisting of stainless steel and nickel-titanium alloys are more preferable, and from the viewpoint of versatility, one or more selected from the group consisting of nickel-titanium alloys are even more preferable. That is, in the medical device according to one embodiment of the present invention, the metal is one or more kinds selected from the group consisting of gold, platinum, silver, copper, nickel, cobalt, titanium, iron, aluminum, tin, stainless steel, nickel-titanium alloys, nickel-cobalt alloys, cobalt-chromium alloys, and zinc-tungsten alloys. According to a more preferred embodiment, the metal is one or more kinds selected from the group consisting of stainless steel and nickel-titanium alloy. According to a further preferred embodiment, the metal is one or more kinds selected from the group consisting of nickel-titanium alloys.

The material that can be used for the above-described base material core portion 10a is not particularly limited, and a material capable of sufficiently exhibiting the function of the base material core portion 10a, that is optimal for the application of the medical device, may be appropriately selected. Examples thereof include, but are not limited to, various polymer materials such as, polyamide resins such as Nylon 6, Nylon 11, Nylon 12, and Nylon 66 (all registered trademarks), polyethylene such as linear low-density polyethylene (LLDPE), low-density polyethylene (LDPE), high-density polyethylene (HDPE), and modified polyethylene, polyolefin resins such as polypropylene, polyester resins such as polyethylene terephthalate, styrene resins such as polystyrene, cyclic polyolefin resins, modified polyolefin resins, epoxy resins, urethane resins, diallyl phthalate resins (allyl resins), polycarbonate resins, fluororesins such as polytetrafluoroethylene (PTFE), expanded polytetrafluoroethylene (ePTFE), polyurethane resins, amino resins (urea resins, melamine resins, benzoguanamine resins), acrylic resins, polyacetal resins, vinyl acetate resins, phenol resins, vinyl chloride resins, silicone resins (silicon resins), polyether resins such as polyether ether ketone (PEEK), and polyimide resins, and inorganic materials such as various ceramic materials.

Alternatively, the base material core portion 10a may be made of a metal material other than the metal material that forms (constitutes) the base material surface layer 10b. Examples of the metal material are not particularly limited, and include the same examples described above. These may be used singly or in combination of two or more kinds thereof.

Preferably, the base material 10 includes or is made of a metal material, or the base material surface layer 10b includes or is made of a metal material. More preferably, the base material 10 is made of a metal material, or the base material surface layer 10b is made of a metal material.

The shape of the base material 10 is not particularly limited, and is appropriately selected from a sheet shape, a film shape, a tubular shape, a shape (for example, Y shape) having a plurality of branch tubes such as a bifurcated shape, a linear shape (wire), a mesh shape, a fiber (yarn) shape, and the like according to the use form.

### <<Functional Layer>>

The functional layer according to the present invention has a polymer including a constituent unit A derived from a bonding monomer having a phosphoric acid group (-OP(=O)(OH)₂, -OP(=O)(OH)O-) or a phosphonic acid group (-P(=O)(OH)₂) and an ethylenically unsaturated group, and a functional polymer chain including a constituent unit B derived from a functional monomer. The polymer is a polymer in which a terminal of the functional polymer chain is bonded to the constituent unit A. The functional layer may have a one-layer form or a laminated form of two or more layers. The functional layer preferably has a one-layer form. The functional layer can be appropriately selected according to the application of the medical device, and examples thereof include, but are not particularly limited to, an antithrombotic layer, an antibacterial layer, an anti-inflammatory layer, and a biodegradable layer. In a case in which the medical device is used in applications where it is in contact with blood, the functional layer is preferably an antithrombotic layer. In the present specification, the "antithrombogenicity" refers to properties of reducing blood coagulation on a surface in contact with blood.

The phosphoric acid group or the phosphonic acid group in the bonding monomer reacts (condensation reaction) with a hydroxyl group (-OH) generated on the surface of the metal base material by a plasma treatment, and forms a phosphate ester bond or a phosphonate ester bond. Thus, the functional layer is firmly immobilized on the metal base material.

The ethylenically unsaturated group in the bonding monomer is a
group that can undergo a polymerization reaction with the functional monomer to be described later. Examples of the ethylenically unsaturated group include an acryloyl group (CH₂=CH-C(=O)-), a methacryloyl group (CH₂=C(CH₃)-C(=O)-), a vinyl group (CH₂=CH-), and an allyl group (CH₂=CH-CH₂-). Among these, the bonding monomer preferably has at least one of an acryloyl group and a methacryloyl group.

Specific examples of the bonding monomer include monomers represented by the following Formulae (I) and (II) .

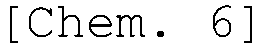

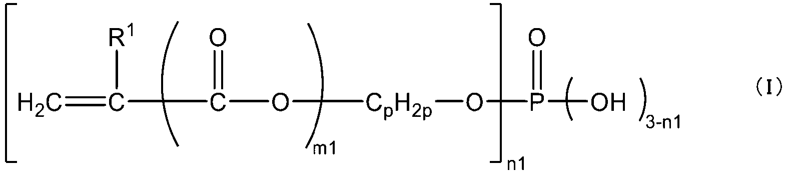

In Formula (I),
R¹ represents a hydrogen atom or a methyl group,
p represents an integer of 0 to 20,
m1 represents 0 or 1, and
n1 represents 1 or 2.

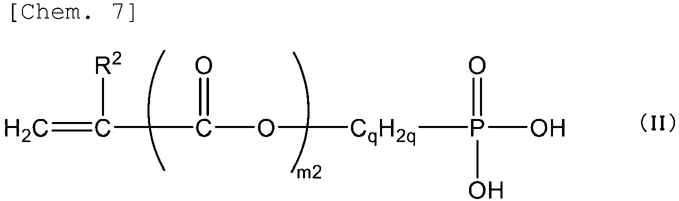

In Formula (II),
R² represents a hydrogen atom or a methyl group,
q represents an integer of 0 to 20, and
m2 represents 0 or 1.

In Formula (I), R¹ is a hydrogen atom or a methyl group, and is preferably a methyl group.

In Formula (I), p is an integer of 0 to 20. From the viewpoint that high functionality (for example, antithrombogenicity) can be imparted, p is preferably an integer of 1 to 20, more preferably an integer of 1 to 10, even more preferably an integer of 1 to 5, and particularly preferably 2.

In Formula (I), m1 is 0 or 1, and is preferably 1.

In Formula (I), n1 is 1 or 2, and is preferably 1.

In Formula (II), R² is a hydrogen atom or a methyl group, and is preferably a methyl group.

In Formula (II), q is an integer of 0 to 20. From the viewpoint that the functionality (for example, antithrombogenicity) can be further improved, q is preferably an integer of 1 to 20, more preferably an integer of 1 to 10, even more preferably an integer of 1 to 5, and particularly preferably 2.

In Formula (II), m2 is 0 or 1, and is preferably 1.

Specific examples of the bonding monomer represented by Formula (I) or (II) include, but are not limited to, 2-(meth)acryloyloxyethyl acid phosphate, bis[2-((meth)acryloyloxy)ethyl] phosphate, 11-phosphonoundecyl (meth)acrylate, vinylphosphonic acid, and allylphosphonic acid. That is, in the medical device according to one embodiment of the present invention, the bonding monomer is one or more kinds selected from 2-(meth)acryloyloxyethyl acid phosphate, bis[2-((meth)acryloyloxy)ethyl] phosphate, 11-phosphonoundecyl (meth)acrylate, vinylphosphonic acid, and allylphosphonic acid. Among these, the bonding monomer is more preferably 2-acryloyloxyethyl acid phosphate and/or 2-methacryloyloxyethyl acid phosphate from the viewpoint that the functionality (for example, antithrombogenicity) can be further improved. The "acid phosphate" is also referred to as "acidic phosphate ester", and refers to a phosphate monoester (P(=O)(OH)₂(OR)) and/or a phosphate diester (P(=O)(OH)(OR)₂) among esters (phosphate esters) of phosphoric acid (P(=O)(OH)₃) and alcohol (R-OH) (that is, the "acid phosphate" refers to a partial ester of phosphoric acid and alcohol).

These bonding monomers may be used singly or in combination of two or more kinds thereof.

The functional monomer is not particularly limited as long as the homopolymer thereof has an intended function (for example, antithrombogenicity, antibacterial properties, anti-inflammatory properties, biodegradability, and the like) of the medical device. In a case in which antithrombogenicity is imparted to the medical device, the functional monomer may be a compound having properties of reducing blood coagulation on a surface where the homopolymer thereof is in contact with blood.

Specific examples of the functional monomer include monomers represented by the following Formulae (III) to (V). Homopolymers derived from these monomers can exhibit excellent antithrombogenicity.

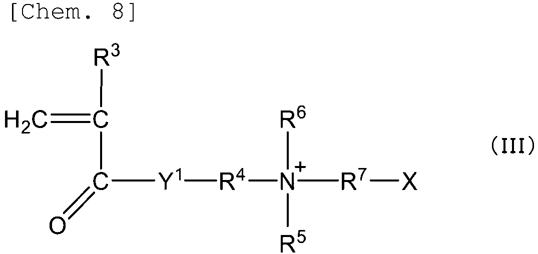

In Formula (III),
R³ represents a hydrogen atom or a methyl group,
R⁴ represents a linear or branched alkylene group having 1 to 6 carbon atoms,
R⁵ and R⁶ each independently represent a linear or branched alkyl group having 1 to 4 carbon atoms,
R⁷ represents a linear or branched alkylene group having 1 to 4 carbon atoms,
X represents -COO⁻ or -SO₃⁻, and
Y¹ represents an oxygen atom or -NH-.

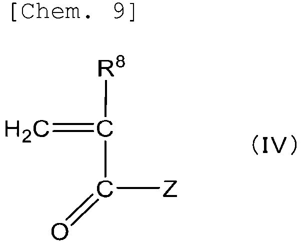

In Formula (IV),
R⁸ represents a hydrogen atom or a methyl group,
Z represents a group represented by Formula: -O-(R⁹O)ₘ-R¹⁰, a group represented by Formula: -O-(CH₂CHOH)ₙ-CH₂OH, or a tetrahydrofurfuryloxy group, in which R⁹ each independently represents a linear or branched alkylene group having 1 to 4 carbon atoms, R¹⁰ represents a linear or branched alkyl group having 1 to 4 carbon atoms, m represents an integer of 1 to 5, and n represents an integer of 1 to 5.

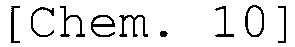

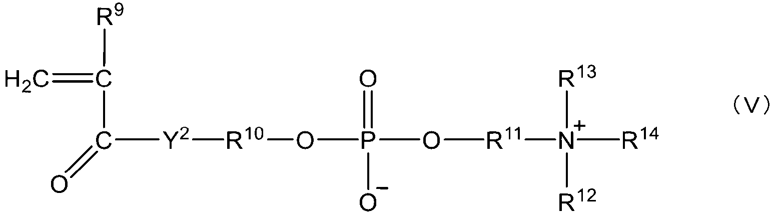

In Formula (V),
R⁹ represents a hydrogen atom or a methyl group,
R¹⁰ and R¹¹ each independently represent a linear or branched alkylene group having 1 to 6 carbon atoms,
R¹² to R¹⁴ each independently represent a linear or branched alkyl group having 1 to 4 carbon atoms, and
Y² represents an oxygen atom or -NH-.

In Formula (III), R³ is a hydrogen atom or a methyl group, and from the viewpoint of obtaining excellent antithrombogenicity, R³ is preferably a methyl group.

In Formula (III), R⁴ is a linear or branched alkylene group having 1 to 6 carbon atoms, and specific examples thereof include a methylene group, an ethylene group, a trimethylene group, a propylene group, a tetramethylene group, a pentamethylene group, and a hexamethylene group. Among these, R⁴ is preferably a linear or branched alkylene group having 1 to 4 carbon atoms, more preferably a methylene group, an ethylene group, or a trimethylene group, and particularly preferably an ethylene group or a trimethylene group from the viewpoint of obtaining excellent antithrombogenicity.

In Formula (III), R⁵ and R⁶ each independently represent a linear or branched alkyl group having 1 to 4 carbon atoms, and specific examples thereof include a methyl group, an ethyl group, a propyl group, an isopropyl group, a butyl group, an isobutyl group, a sec-butyl group, and a tert-butyl group. Among these, R⁵ and R⁶ are each independently preferably a linear or branched alkyl group having 1 to 3 carbon atoms, more preferably an alkyl group having 1 or 2 carbon atoms (methyl group, ethyl group), and particularly preferably a methyl group from the viewpoint of obtaining excellent antithrombogenicity.

In Formula (III), R⁷ is a linear or branched alkylene group having 1 to 4 carbon atoms, and specific examples thereof include a methylene group, an ethylene group, a trimethylene group, a propylene group, and a tetramethylene group. Among these, R⁷ is preferably a methylene group, an ethylene group, or a trimethylene group, and more preferably a methylene group from the viewpoint of obtaining excellent antithrombogenicity.

In Formula (III), X is a carboxybetaine zwitterionic monomer (-COO⁻) or a sulfobetaine zwitterionic monomer (-SO₃⁻). X is preferably -COO⁻ from the viewpoint of obtaining excellent antithrombogenicity.

In Formula (III), Y¹ is an oxygen atom or -NH-, and from the viewpoint of obtaining excellent antithrombogenicity, Y¹ is preferably an oxygen atom.

In Formula (IV), R⁸ is a hydrogen atom or a methyl group, and from the viewpoint of obtaining excellent antithrombogenicity, R⁸ is preferably a hydrogen atom.

In Formula (IV), in a case in which Z is a group represented by Formula: -O-(R⁹O)ₘ-R¹⁰, R⁹ is each independently a linear or branched alkylene group having 1 to 4 carbon atoms, and specific examples thereof include a methylene group, an ethylene group, a trimethylene group, a propylene group, and a tetramethylene group. Among these, R⁹ is each independently more preferably a methylene group, an ethylene group, or a trimethylene group, particularly preferably an ethylene group or a trimethylene group, and most preferably an ethylene group from the viewpoint of obtaining excellent antithrombogenicity. In a case in which a plurality of -R⁹O- are present (m is 2 or more), each -R⁹O- may be the same or different.

In Formula (IV), in a case in which Z is a group represented by Formula: -O-(R⁹O)ₘ-R¹⁰, R¹⁰ is a linear or branched alkyl group having 1 to 4 carbon atoms, and specific examples thereof include linear or branched alkyl groups such as a methyl group, an ethyl group, a propyl group, an isopropyl group, a butyl group, an isobutyl group, a sec-butyl group, and a tert-butyl group. Among these, R¹⁰ is preferably a linear or branched alkyl group having 1 to 3 carbon atoms, more preferably an alkyl group having 1 or 2 carbon atoms (methyl group, ethyl group), and particularly preferably a methyl group from the viewpoint of obtaining excellent antithrombogenicity.

In Formula (IV), in a case in which Z is a group represented by Formula: -O-(R⁹O)ₘ-R¹⁰, m represents the number of (-R⁹O-) groups and is an integer of 1 to 5. m is preferably an integer of 1 to 3, more preferably 1 or 2, and particularly preferably 1 from the viewpoint of improving antithrombogenicity.

In Formula (IV), in a case in which Z is a group represented by Formula: -O-(CH₂CHOH)ₙ-CH₂OH, n represents the number of (-CH₂CHOH-) groups and is an integer of 1 to 5. m is preferably an integer of 1 to 3, more preferably 1 or 2, and particularly preferably 1 from the viewpoint of improving antithrombogenicity.

In Formula (IV), Z is a group represented by -O-(R⁹O)ₘ-R¹⁰, a group represented by -O-(CH₂CHOH)ₙ-CH₂OH, or a tetrahydrofurfuryloxy group, and from the viewpoint of obtaining excellent antithrombogenicity, Z is preferably a group represented by -O-(R⁹O)ₘ-R¹⁰.

In Formula (V), R⁹ is a hydrogen atom or a methyl group, and from the viewpoint of obtaining excellent antithrombogenicity, R⁹ is preferably a methyl group.

In Formula (V), R¹⁰ and R¹¹ are each independently a linear or branched alkylene group having 1 to 6 carbon atoms. Here, R¹⁰ and R¹¹ may be the same or different. Specific examples of the linear or branched alkylene group having 1 to 6 carbon atoms include a methylene group, an ethylene group, a trimethylene group, a propylene group, a tetramethylene group, a pentamethylene group, and a hexamethylene group. Among these, R¹⁰ and R¹¹ are each independently preferably a linear or branched alkylene group having 1 to 3 carbon atoms, more preferably a methylene group or an ethylene group, and particularly preferably an ethylene group from the viewpoint of obtaining excellent antithrombogenicity.

In Formula (V), R¹² to R¹⁴ each independently represent a linear or branched alkyl group having 1 to 4 carbon atoms. Here, R¹² to R¹⁴ may be the same or different. Specific examples of the alkyl group having 1 to 4 carbon atoms include a methyl group, an ethyl group, a propyl group, an isopropyl group, a butyl group, an isobutyl group, a sec-butyl group, and a tert-butyl group. Among these, R¹² to R¹⁴ are each independently preferably a linear or branched alkyl group having 1 to 3 carbon atoms, more preferably an alkyl group having 1 or 2 carbon atoms (methyl group, ethyl group), and particularly preferably a methyl group from the viewpoint of obtaining excellent antithrombogenicity.

In Formula (V), Y² is an oxygen atom or -NH-, and from the viewpoint of obtaining excellent antithrombogenicity, Y² is preferably an oxygen atom.

The monomer represented by Formula (IV) has cell adhesiveness to vascular endothelial cells, in addition to antithrombogenicity. Thus, in a case in which one or more kinds selected from the monomers represented by Formula (IV) are used as an antithrombotic monomer, a functional layer having both antithrombogenicity and adhesiveness to vascular endothelial cells can be formed on the surface of the metal base material. Therefore, for example, in a medical device such as a stent, a stent graft, or a covered stent, the surface of the metal base material that is exposed to blood is easily covered with endothelial cells, and the risk of clot formation can be further reduced.

Among the zwitterionic monomers represented by Formula (III), examples of the zwitterionic monomer having a carboxybetaine skeleton (carboxybetaine zwitterionic monomer; X=-COO⁻) include N-(meth)acryloyloxymethyl-N,N-dimethylammonium-α-N-methylcarboxybetaine, N-(meth)acryloyloxyethyl-N,N-dimethylammonium-α-N-methylcarboxybetaine, N-(meth)acryloyloxypropyl-N,N-dimethylammonium-α-N-methylcarboxybetaine, N-(meth)acryloyloxymethyl-N,N-diethylammonium-α-N-methylcarboxybetaine, N-(meth)acryloyloxyethyl-N,N-diethylammonium-α-N-methylcarboxybetaine, and N-(meth)acryloyloxypropyl-N,N-diethylammonium-α-N-methylcarboxybetaine. Among these, N-(meth)acryloyloxyethyl-N,N-dimethylammonium-α-N-methylcarboxybetaine is preferable, and N-methacryloyloxyethyl-N,N-dimethylammonium-α-N-methylcarboxybetaine (CBA) is more preferable from the viewpoint of obtaining excellent antithrombogenicity.

Among the zwitterionic monomers represented by Formula (III), examples of the zwitterionic monomer having a sulfobetaine skeleton (sulfobetaine zwitterionic monomer; X=-SO₃⁻) include [2-((meth)acryloyloxy)ethyl]dimethyl- (3-sulfopropyl)ammonium hydroxide, [2-((meth)acryloyloxy)ethyl]dimethyl-(3-sulfobutyl)ammonium hydroxide, [2-((meth)acryloyloxy)ethyl]diethyl-(3-sulfopropyl)ammonium hydroxide, and [2-((meth)acryloyloxy)ethyl]diethyl-(3-sulfobutyl)ammonium hydroxide. Among these, [2-((meth)acryloyloxy)ethyl]dimethyl-(3-sulfopropyl)ammonium hydroxide is preferable.

Specific examples of the monomer represented by Formula (IV) include, but are not limited to, methoxymethyl acrylate, 2-methoxyethyl acrylate (MEA), 3-methoxypropyl acrylate (MC3A), ethoxymethyl acrylate, ethoxyethyl acrylate (EEA), 2-(2-ethoxyethoxy)ethyl acrylate (EEEA), ethoxypropyl acrylate, ethoxybutyl acrylate, propoxymethyl acrylate, butoxyethyl acrylate, methoxybutyl acrylate, tetrahydrofurfuryl acrylate (THFA), methoxymethyl methacrylate, methoxyethyl methacrylate, ethoxymethyl methacrylate, ethoxyethyl methacrylate, 2-(2-ethoxyethoxy)ethyl methacrylate, propoxymethyl methacrylate, butoxyethyl methacrylate, tetrahydrofurfuryl methacrylate, and glycerol (meth)acrylate. Among these, 2-methoxyethyl acrylate (MEA), 3-methoxypropyl acrylate (MC3A), and tetrahydrofurfuryl acrylate (THFA) are preferable, and 2-methoxyethyl acrylate (MEA) and 3-methoxypropyl acrylate (MC3A) are more preferable.

Specific examples of the monomer represented by Formula (V) include 2-(meth)acryloyloxyethyl phosphorylcholine. Among these, 2-methacryloyloxyethyl phosphorylcholine (MPC) is more preferable.

That is, in the medical device according to one embodiment of the present invention, the functional monomer is one or more kinds selected from N-(meth)acryloyloxymethyl-N,N-dimethylammonium-α-N-methylcarboxybetaine, N-(meth)acryloyloxyethyl-N,N-dimethylammonium-α-N-methylcarboxybetaine, N-(meth)acryloyloxypropyl-N,N-dimethylammonium-α-N-methylcarboxybetaine, N-(meth)acryloyloxymethyl-N,N-diethylammonium-α-N-methylcarboxybetaine, N-(meth)acryloyloxyethyl-N,N-diethylammonium-α-N-methylcarboxybetaine, N-(meth)acryloyloxypropyl-N,N-diethylammonium-α-N-methylcarboxybetaine, methoxymethyl acrylate, 2-methoxyethyl acrylate, 3-methoxypropyl acrylate, ethoxymethyl acrylate, ethoxyethyl acrylate, 2-(2-ethoxyethoxy)ethyl acrylate, ethoxypropyl acrylate, ethoxybutyl acrylate, propoxymethyl acrylate, butoxyethyl acrylate, methoxybutyl acrylate, tetrahydrofurfuryl acrylate, methoxymethyl methacrylate, methoxyethyl methacrylate, ethoxymethyl methacrylate, ethoxyethyl methacrylate, 2-(2-ethoxyethoxy)ethyl methacrylate, propoxymethyl methacrylate, butoxyethyl methacrylate, tetrahydrofurfuryl methacrylate, glycerol (meth)acrylate, and 2-methacryloyloxyethyl phosphorylcholine.

According to a more preferred embodiment, the functional monomer is one or more kinds selected from the group consisting of methoxymethyl acrylate, 2-methoxyethyl acrylate (MEA), 3-methoxypropyl acrylate (MC3A), ethoxymethyl acrylate, ethoxyethyl acrylate (EEA), 2-(2-ethoxyethoxy)ethyl acrylate (EEEA), ethoxypropyl acrylate, ethoxybutyl acrylate, propoxymethyl acrylate, butoxyethyl acrylate, methoxybutyl acrylate, tetrahydrofurfuryl acrylate (THFA), methoxymethyl methacrylate, methoxyethyl methacrylate, ethoxymethyl methacrylate, ethoxyethyl methacrylate, 2-(2-ethoxyethoxy)ethyl methacrylate, propoxymethyl methacrylate, butoxyethyl methacrylate, tetrahydrofurfuryl methacrylate, and glycerol (meth)acrylate.

These functional monomers may be used singly or in combination of two or more kinds thereof. That is, the functional polymer chain including the constituent unit B derived from the functional monomer may be a homopolymer composed of one functional monomer alone, or a copolymer composed of two or more kinds of the functional monomers. In a case in which two or more kinds are used, the form of the constituent unit B may be a block copolymer or a random copolymer.

In the polymer according to the present invention, the ratio of the number of the constituent units B to the number of the constituent units A is not particularly limited. However, since sufficient functionality (for example, antithrombogenicity) is exhibited, the ratio is preferably 30 or more, more preferably 30 or more and 200 or less, and even more preferably 30 or more and 150 or less. That is, in the medical device according to one embodiment of the present invention, the ratio of the number of the constituent units B to the number of the constituent units A in the polymer is 30 or more. By controlling the number of moles of the functional monomer to be reacted (brought into contact) with the base material after the bonding monomer is reacted, and the reaction time of the functional monomer with the base material, a polymer in which a predetermined number of constituent units B are connected to one constituent unit A can be obtained.

The terminal of the polymer according to the present invention is not particularly limited, and can be appropriately determined according to the kind of the monomer used. In one embodiment, the terminal (* in Fig. 1) of the polymer according to the present invention is a hydrogen atom. In addition, in a case in which the polymer according to the present invention is prepared by a radical polymerization method, the terminal (* in Fig. 1) of the polymer can be sealed with a constituent unit (functional group) derived from a polymerization initiator. In the latter form (form in which the terminal of the polymer is sealed with a functional group derived from a polymerization initiator), the terminal of the polymer may be a carboxyl group.

The weight average molecular weight of the polymer according to the present invention is thousands to millions, preferably 10,000 to 5,000,000. In the present specification, as the "weight average molecular weight", a value measured by gel
permeation chromatography (GPC) using polystyrene as a standard substance and tetrahydrofuran (THF) as a mobile phase is adopted. The molecular weight of the copolymer can also be calculated from the kind of the constituent units and the number of the constituent units.

The thickness (dry film thickness) of the functional layer is, for example, 0.1 to 10 µm, preferably 0.5 to 5 µm, and more preferably about 1 to 3 µm.

### [Method for Manufacturing Medical Device]

Another aspect of the present invention also provides a method for manufacturing the medical device. That is, one embodiment of the present invention is a method for manufacturing the medical device (in the present specification, also simply referred to as the "method for manufacturing a medical device according to the present invention"), including: irradiating at least a part of a surface of the base material (metal base material) with plasma; bringing the bonding monomer into contact with the base material after irradiation with the plasma; and bringing the functional monomer into contact with the base material after contact with the bonding monomer to form the functional polymer chain on the surface of the base material via the constituent unit A derived from the bonding monomer.

Hereinafter, preferred embodiments of the steps performed in the method for manufacturing a medical device according to the present invention will be described. Note that the method for manufacturing a medical device according to the present invention is not limited to the following embodiments.

### <<Plasma Irradiation Step (plasma treatment step)>>

In this step, at least a part of a surface of the base material (metal base material) is irradiated with plasma (plasma treatment is performed). Therefore, a hydroxyl group (-OH) can be generated on the surface of the metal base material. Then, by bringing the bonding monomer into contact with the surface of the metal base material, a phosphoric acid group or a phosphonic acid group contained in the bonding monomer reacts (condensation reaction) with the hydroxyl group (-OH) formed on the surface of the base material, and a phosphate ester bond or a phosphonate ester bond can be formed. As a result, the polymer according to the present invention is firmly immobilized on the metal base material, and thus it is possible to manufacture a medical device capable of maintaining functionality (for example, antithrombogenicity) for a long period of time.

Since a desired plasma treatment can be performed even on a narrow inner surface of a small-diameter artificial blood vessel or the like, the plasma irradiation step is preferably performed through an ionized gas plasma treatment.

Before the surface of the metal base material is irradiated with the ionized gas plasma, the surface of the metal base material may be polished and/or cleaned by an appropriate method. That is, before the hydroxyl group (-OH) is generated on the surface of the metal base material by ionized gas plasma irradiation, it is preferable to smooth the surface of the metal base material or to remove oil and fat, dirt, and the like adhering to the surface of the metal base material. The polishing method is not particularly limited, and a known polishing method (for example, chemical polishing, electrolytic polishing, mechanical polishing) can be appropriately adopted. Among these, chemical polishing is preferable to perform the polishing since it is suitable for polishing fine portions. In addition, the cleaning method is also not particularly limited, and can be performed by cleaning with an appropriate solvent (for example, ultrasonic cleaning, a method for performing immersion in a cleaning solvent, a method for pouring a cleaning solvent, or the like).

The pressure condition in the plasma treatment is not particularly limited, and can be either reduced pressure or atmospheric pressure. From the viewpoints that plasma gas irradiation can be performed from free angles, that device downsizing is possible since no vacuum device is required, that a space-saving and low-cost system configuration can be realized, and that it is also economically advantageous, the plasma treatment is preferably performed under atmospheric pressure. In a case in which a plasma irradiation nozzle applies a plasma gas while rotating around an object to be treated, such as an artificial blood vessel, the entire circumference of the object to be treated can be uniformly plasma-treated without unevenness.

The ionized gas that can be used for the plasma treatment is one or more gases selected from the group consisting of helium, neon, argon, krypton, air, oxygen, carbon dioxide, carbon monoxide, water vapor, nitrogen, hydrogen, and the like. The plasma treatment conditions (irradiation time, LF output, applied current, gas flow rate, gap between electrodes, plasma irradiation distance) are not particularly limited, and can be appropriately selected according to bonding properties (ease of immobilization) between the bonding monomer and the metal base material, the kind and area of the metal base material to be used, and the like.

As an example, the irradiation time in the plasma treatment is preferably longer than 10 seconds, more preferably 30 seconds or longer and 10 minutes or shorter, and particularly preferably 1 to 5 minutes. Under such conditions, a sufficient amount of the bonding monomer can be bonded (immobilized) to the surface of the metal base material.

As an example, the LF output in the plasma treatment is preferably 100 to 300 W, more preferably 150 to 250 W, and even more preferably 200 W.

As an example, the gas flow rate in the plasma treatment is preferably 5 to 20 sccm, more preferably 10 to 20 sccm, and even more preferably 15 sccm.

The temperature of the object to be treated (metal base material) in the plasma treatment is not particularly limited, and may be room temperature. Alternatively, the object may be heated or cooled. The plasma treatment is preferably performed at a temperature at which a heating device or a cooling device is unnecessary (for example, 5 to 35°C) from an economical point of view.

A plasma irradiation device (system) that can be used for the plasma treatment is not particularly limited. Examples thereof include a plasma irradiation device (system) including a plasma generation tube that generates plasma by introducing gas molecules and exciting the gas molecules, and an electrode that excites the gas molecules in the plasma generation tube, in which plasma is emitted from one end of the plasma generation tube, but are not limited to such a configuration (system) at all. Examples thereof include devices adopting a high frequency induction method, a capacity-coupled electrode method, a corona discharge electrode-plasma jet method, a parallel plate type, a remote plasma type, an atmospheric pressure plasma type, a low pressure plasma type, and an ICP type high density plasma type. In addition, an ionized gas plasma irradiation device (system) suitable for irradiation on stent grafts, covered stents, stents, guide wires, cardiac pacemakers, and the like, particularly, a plasma irradiation device (system) at atmospheric pressure can be used from among those already commercially available. Specifically, a plasma irradiation device manufactured by Tri-Star Technologies Inc.: DURADYNE (product name or trade name), a plasma irradiation device manufactured by Diener electronic GmbH & Co. KG: PLASMABEAM, Pico Full PC, or the like can be used, but the plasma irradiation device is not limited thereto at all.

### <<Bonding Monomer Contact Step>>

In this step, the bonding monomer is brought into contact with the base material after irradiation with the plasma. Therefore, the phosphoric acid group or the phosphonic acid group contained in the bonding monomer reacts (condensation reaction) with the hydroxyl group (-OH) formed on the surface of the base material, and a phosphate ester bond or a phosphonate ester bond can be formed.

In this step, as a specific method for bringing the bonding monomer into contact with the base material after irradiation with the plasma, either a method for bringing the bonding monomer into contact with the base material as it is, or a method for preparing a bonding monomer solution in which the bonding monomer is dissolved in a solvent and then bringing the bonding monomer solution into contact with the base material may be used. From the viewpoint of forming a more uniform functional layer, the latter method for bringing the bonding monomer solution into contact with the base material is preferably adopted.

The solvent used for preparing the bonding monomer solution is not particularly limited, and is appropriately selected according to the kind of the bonding monomer (and other components, if used). Water (reverse osmosis membrane water (RO water), pure water, ion-exchanged water, distilled water, and the like); alcohol-based solvents such as methanol, ethanol, isopropyl alcohol, and butanol; and organic solvents such as dichloromethane, chloroform, carbon tetrachloride, tetrahydrofuran (THF), dimethyl sulfoxide, N,N-dimethylformamide (DMF), acetone, dioxane, and benzene are preferably used from a viewpoint of high solubility. These solvents may be used singly or in mixture of two or more kinds thereof (in the form of mixed solvent).

The concentration of the bonding monomer in the bonding monomer solution is not particularly limited. For example, the concentration is preferably 0.0001 to 10 mass%, more preferably 0.0001 to 1 mass%, and particularly preferably 0.0003 to 1 mass%. In a case in which the concentration of the bonding monomer is within the above range, the functional layer to be obtained can exhibit a sufficient function.

Next, the bonding monomer solution prepared as described above is brought into contact with the base material after irradiation with the plasma. Here, as a specific "contact" method, a method that has been known, such as a method for applying the bonding monomer solution to the surface of the base material or a method for immersing the base material in the bonding monomer solution, can be appropriately adopted. Among these, an immersion method that is easy to operate is preferably used.

In a case in which the functional layer is formed on a narrow inner surface of a stent graft, a covered stent, a stent, a guide wire, a cardiac pacemaker, or the like, the metal base material may be immersed in the bonding monomer solution and subjected to defoaming by reducing the pressure in the system. Defoaming under reduced pressure makes it possible to quickly permeate the solution into the narrow inner surface and promote the bonding of the bonding monomer to the surface of the base material.

Alternatively, the contact by the immersion method may be performed while the bonding monomer solution is stirred. This makes it possible to further promote the contact between the bonding monomer solution and the object to be treated. The stirring can be performed by stirring using a stirrer, or using a known device such as an orbital shaker, a rotary shaker, or a paint shaker. In addition, the stirring conditions are not particularly limited. The stirring speed is, for example, 200 to 600 rpm, and preferably 300 to 400 rpm from the viewpoint of more efficient contact with the object to be treated. From the same viewpoint, the stirring time is, for example, 0.5 to 24 hours, and preferably 1 to 12 hours. The temperature of the bonding monomer solution during contact by the immersion method may be about room temperature (for example, 20 to 40°C), but heating (for example, 40 to 60°C) may be performed.

In addition, in a case in which the functional layer is formed only on a part of the base material, the bonding monomer can be bonded to a desired surface site of the base material by immersing only a part of the base material in the bonding monomer solution.

In a case in which it is difficult to immerse only a part of the metal base material in the bonding monomer solution, the bonding monomer can be bonded to a desired surface site of the base material by previously protecting (covering or the like) a surface portion of the base material on which the functional layer does not need to be formed with an appropriate detachable member or material (configured to be attached or detached), immersing the base material in the bonding monomer solution, and then detaching the protective member (material) on the surface portion of the base material on which the functional layer does not need to be formed. In the present invention, there is no limitation to these forming methods at all, and the bonding monomer can be bonded by appropriately using a method that has been known. For example, in a case in which it is difficult to immerse only a part of the metal base material in the bonding monomer solution, another coating method (for example, a method for applying the bonding monomer solution to a predetermined surface portion of the medical device using an application device such as a spray device, a bar coater, a die coater, a reverse coater, a comma coater, a gravure coater, a spray coater, or a doctor knife) may be applied instead of the immersion method. In a case in which both an outer surface and an inner surface of the cylindrical tool need to have a functional layer due to the structure of the medical device, the immersion method (dipping method) is preferably used from the viewpoint that the bonding monomer can be bonded to both the outer surface and the inner surface at one time.

In this step, the base material after contact with the bonding monomer may be cleaned if necessary. The cleaning method is not particularly limited, and can be performed by cleaning with an appropriate solvent (for example, ultrasonic cleaning, a method for performing immersion in a cleaning solvent, a method for pouring a cleaning solvent, or the like). The cleaning time is not particularly limited, and is preferably 1 to 60 minutes. The cleaning treatment may be repeated a plurality of times (for example, 2 to 5 times) if necessary.

### <<Functional Monomer Contact Step>>

In this step, the functional monomer (antithrombotic monomer) is brought into contact with the base material after contact with the bonding monomer. Therefore, the functional monomer is bonded to the ethylenically unsaturated group contained in the constituent unit A derived from the bonding monomer bonded to the base material, and the remaining functional monomer is graft-polymerized with the functional monomer bonded to the ethylenically unsaturated group, so that a functional polymer chain is formed on the surface of the base material via the constituent unit A derived from the bonding monomer. That is, the functional layer is formed on the surface of the base material.

In this step, as a specific method for bringing the functional monomer into contact with the base material after contact with the bonding monomer, either a method for bringing the functional monomer into contact with the base material after contact with the bonding monomer as it is,
or a method for preparing a functional monomer solution in which the functional monomer is dissolved in a solvent and then bringing the functional monomer solution into contact with the base material after contact with the bonding monomer may be used. From the viewpoint of forming a more uniform functional layer, the latter method for bringing the functional monomer solution into contact with the base material after contact with the bonding monomer is preferably adopted.

The solvent (polymerization solvent) used for preparing the functional monomer solution is not particularly limited as long as it can dissolve the functional monomer and an appropriate polymerization initiator, and examples thereof include one or more kinds selected from water (reverse osmosis membrane water (RO water), pure water, ionexchanged water, distilled water, and the like); alcohols such as ethanol, isopropanol, n-propanol, n-butanol, isobutanol, sec-butanol, t-butanol, ethylene glycol, diethylene glycol, propylene glycol, and dipropylene glycol; organic solvents such as chloroform, tetrahydrofuran, acetone, dioxane, and benzene, and the like, but are not limited thereto. The concentration of the functional monomer contained in the functional monomer solution is not particularly limited, and by setting the concentration relatively high, the weight average molecular weight of the polymer to be obtained can be increased. As an example, the concentration (total concentration) of the functional monomer in the functional monomer solution is preferably 10 mass% or more, and more preferably 15 mass% or more. In addition, the upper limit of the concentration (total concentration) of the functional monomer is not particularly limited, and is, for example, a saturated concentration or less, e.g., 70 mass% or less, and preferably 50 mass% or less.

In this step, by bringing the functional monomer into contact with the base material after contact with the bonding monomer, the functional monomer is bonded to the ethylenically unsaturated group contained in the constituent unit A derived from the bonding monomer bonded to the base material, and the remaining functional monomer is graft-polymerized with the functional monomer bonded to the ethylenically unsaturated group. The specific polymerization method is not particularly limited, and for example, a polymerization method that has been known, such as a radical polymerization method or a polymerization method using a macroinitiator, can be adopted. In addition, examples of the polymerization reaction method include (i) a method in which a polymerization reaction solution obtained by dissolving a functional monomer and an appropriate polymerization initiator in a polymerization solvent is heated to carry out a polymerization reaction; and (ii) a method in which a solution obtained by dissolving a functional monomer in a polymerization solvent is mixed with a separately prepared polymerization initiator solution to prepare a polymerization reaction solution, and a polymerization reaction is carried out. From the viewpoint of ease of operation, the method (i) is preferable.

In addition, the polymerization reaction solution may be subjected to a degassing treatment before the polymerization reaction is carried out. The degassing treatment can be performed, for example, by bubbling with an inert gas such as a nitrogen gas or an argon gas for about 5 minutes to 1 hour.

In addition, the polymerization initiator used in this case is not particularly limited, and a known polymerization initiator can be used. From the viewpoint of excellent polymerization stability, a radical polymerization initiator is preferably used, and specific examples thereof include persulfates such as potassium persulfate (KPS), sodium persulfate, and ammonium persulfate; peroxides such as hydrogen peroxide, t-butyl peroxide, methyl ethyl ketone peroxide, 3-hydroxy-1,1-dimethylbutyl peroxyneodecanoate, α-cumyl peroxyneodecanoate, 1,1,3,3-tetrabutyl peroxyneodecanoate, t-butyl peroxyneodecanoate, t-butyl peroxyneoheptanoate, t-butyl peroxypivalate, t-amyl peroxyneodecanoate, t-amyl peroxypivalate, di(2-ethylhexyl)peroxydicarbonate, and di(s-butyl)peroxydicarbonate; and azo compounds such as azobisisobutyronitrile (AIBN), 2,2'-azobis(4-methoxy-2,4-dimethylvaleronitrile), 2,2'-azobis(2,4-dimethylvaleronitrile), 2,2'-azobis[2-(2-imidazoline-2-yl)propane]dihydrochloride, 2,2'-azobis[2-(2-imidazoline-2-yl)propane]disulfate dihydrate, 2,2'-azobis(2-methylpropionamidine)dihydrochloride, 2,2'-azobis[N-(2-carboxyethyl)-2-methylpropionamidine)]hydrate, 2,2'-azobis[N-(2-carboxyethyl)-2-methylpropionamidine)]tetrahydrate, and azobiscyanovaleric acid. The polymerization initiators may be used singly or in combination of two or more kinds thereof.

In addition, for example, a reducing agent such as sodium sulfite, sodium bisulfite, or ascorbic acid may be used in combination with the radical polymerization initiator as a redox initiator. The blending amount (addition amount) of the polymerization initiator is preferably 0.01 to 5 mass%, and more preferably 0.05 to 3 mass% with respect to 100 mass% of the total of the functional monomers. Alternatively, the blending amount (addition amount) of the polymerization initiator is preferably 0.01 to 5 mol, and more preferably 0.05 to 3 mol with respect to 100 mol of the total of the functional monomers. With such a blending amount (addition amount) of the polymerization initiator, the polymerization reaction can be efficiently advanced.

In addition, in a case in which the method (ii) is used, the solvent in which the polymerization initiator is previously dissolved (solvent of the polymerization initiator solution) is not particularly limited as long as it can dissolve the polymerization initiator, and examples thereof include the same solvents as the polymerization solvents. The solvent of the polymerization initiator solution may be the same as or different from the polymerization solvent, but is preferably the same as the polymerization solvent in consideration of ease of polymerization control and the like.

By heating the polymerization reaction solution prepared as described above, the ethylenically unsaturated group contained in the constituent unit A derived from the bonding monomer bonded to the base material can be polymerized with the functional monomer. Here, as the polymerization method, for example, a known polymerization method such as anionic polymerization or cationic polymerization can be adopted other than the above-described radical polymerization, and the radical polymerization is preferably used in consideration of ease of operation.

The polymerization conditions are not particularly limited as long as the ethylenically unsaturated group contained in the constituent unit A derived from the bonding monomer bonded to the base material can be polymerized with the functional monomer. As an example, the polymerization temperature is preferably 30 to 150°C, and more preferably 40 to 100°C. In addition, the polymerization time is preferably 30 minutes to 30 hours, and more preferably 1 to 10 hours. Under such conditions, the polymerization reaction can be efficiently advanced.

In addition, in the polymerization, a chain transfer agent, a polymerization rate adjusting agent, a surfactant, and other additives may be appropriately used if necessary.

The environment in which the polymerization reaction is carried out is not particularly limited, and it is possible to carry out the polymerization reaction under an air atmosphere, an inert gas atmosphere such as a nitrogen gas or an argon gas, or the like. The polymerization reaction is preferably carried out under a nitrogen gas atmosphere. During the polymerization reaction, the reaction solution may be stirred.

In this step, the base material after contact with the functional monomer may be cleaned if necessary. The cleaning method is not particularly limited, and can be performed by cleaning with an appropriate solvent (for example, ultrasonic cleaning, a method for performing immersion in a cleaning solvent, a method for pouring a cleaning solvent, or the like). The cleaning time is not particularly limited, and is preferably 1 to 60 minutes. The cleaning treatment may be repeated a plurality of times (for example, 2 to 5 times) if necessary.

Through the above method, a medical device having excellent functionality (for example, antithrombogenicity) is manufactured.

### [Application of Medical Device]

The medical device according to the present invention has excellent functionality (for example, antithrombogenicity). Examples of the medical device
according to the present invention include in-vivo implantable artificial organs and treatment instruments, extracorporeal circulation type artificial organs, catheters, and guide wires. Among these, the medical device is preferably an intravascular indwelling device, and specific examples thereof include stent grafts, covered stents, stents, guide wires, and cardiac pacemakers. That is, the medical device according to one embodiment of the present invention is a stent graft, a covered stent, a stent, a guide wire, or a cardiac pacemaker. The following medical devices are also included. Specific examples thereof include implantable medical devices such as artificial windpipes, artificial joints, and artificial pericardiums, artificial organ systems such as artificial heart systems, artificial lung systems, artificial heartlung systems, artificial kidney systems, artificial liver systems, and immunomodulation systems, catheters that are inserted into or indwelled in blood vessels such as indwelling needles, IVH catheters, liquid medicine administering catheters, thermodilution catheters, angiography catheters, and catheters and dilators or introducers for vasodilatation, and catheters that are inserted into or indwelled in biological tissues other than blood vessels such as various suction catheters and drainage catheters, including guide wires and stylet for the above catheters, gastric tube catheters, feeding catheters, tubes for tubal feeding (ED), urethral catheters, urinary catheters, balloon catheters, and intratracheal suction catheters.

### Examples

The effects of the present invention will be described with reference to the following examples and comparative examples. Note that the technical scope of the present invention is not limited only to the following examples. In the following examples, the notation "parts" or "%" may be used, but unless otherwise specified, it represents "parts by mass" or "mass%". Unless otherwise specified, each operation was performed at room temperature (25°C).

### [Example 1]

### <Production of NiTi-PHA-CBA>

### (1) Preparation of NiTi Base Material

Nickel-titanium (NiTi) (manufactured by SAES Getters S.p.A., thickness: 0.5 mm) was cut into a size of 1.0 cm×2.0 cm. The cut NiTi and 300 mL of a MicroClean BS polishing solution (manufactured by RD Chemical Company) were put in a 500 mL beaker and cleaned with an ultrasonic cleaner for 3 minutes. This operation was repeated 10 times. The NiTi after cleaning was put in distilled water and washed in the water with an ultrasonic cleaner for 3 minutes. This operation was repeated three times to remove the polishing solution adhering to the surface of NiTi. The NiTi after water washing was dried at room temperature, and thus a NiTi base material (NiTi (not plasma-treated)) was obtained.

### (2) Plasma Treatment of NiTi Base Material

The NiTi base material was set in a plasma irradiation device (Pico Full PC, manufactured by Diener electronic GmbH & Co. KG, low pressure type), and plasma irradiation (argon ionized gas plasma irradiation) was performed on both surfaces for 3 minutes under conditions of pressure: 0.3 mbar, power: 50%, introduction gas: 100% Ar gas, LF output: 200 W, and gas flow rate: 15 sccm. The NiTi base material after plasma irradiation was taken out in the atmosphere and left for 10 to 30 minutes. Therefore, a NiTi base material (NiTi (after plasma treatment)) whose surface was plasma-treated was obtained.

### (3) Bonding of 2-Methacryloyloxyethyl Acid Phosphate (PHA)

16.6 µL (0.1 mmol) of 2-methacryloyloxyethyl acid phosphate (PHA) (LIGHT ESTER P-1M, manufactured by Kyoeisha Chemical Co., Ltd.) and 10 mL of ethanol were put in a glass bottle to dissolve the reagent. In this solution, the NiTi base material was put to carry out a reaction at room temperature overnight. Thereafter, the NiTi base material was taken out and left in ethanol for 1 hour to remove the unreacted PHA, and then dried at room temperature. Therefore, a NiTi base material (NiTi-PHA) having PHA bonded thereto was obtained.

### (4) Polymerization of N-Methacryloyloxyethyl-N,N-Dimethylammonium-α-N-Methylcarboxybetaine (CBA)

1.97 g (9.15 mmol) of N-methacryloyloxyethyl-N,N-dimethylammonium-α-N-methylcarboxybetaine (CBA, monomer concentration: 20 mass%, manufactured by OSAKA ORGANIC CHEMICAL INDUSTRY LTD.), 1.9 mg of 2,2'-azobis(4-methoxy-2,4-dimethylvaleronitrile) (V-70, manufactured by FUJIFILM Wako Pure Chemical Corporation), and 10 mL of ethanol were put in a glass bottle, and the resulting mixture was stirred at room temperature until all the reagents were dissolved. The reagent solution in the glass bottle was transferred to an eggplant flask, the NiTi base material (NiTi-PHA) having PHA bonded thereto was put in the reagent solution, a nitrogen gas was introduced for 10 minutes, and the gas in the system was replaced with a nitrogen gas. The eggplant flask was covered with a rubber plug. The eggplant flask was put in a water bath at 37°C to carry out a reaction for 4 hours. Thereafter, the NiTi base material was taken out and cleaned by being immersed in ethanol to remove the unreacted substances. Therefore, a NiTi base material (NiTi-PHA-CBA) in which CBA was polymerized via PHA was obtained.

### [Example 2]

### <Production of NiTi-PHA-MEA>

### (4) Polymerization of 2-Methoxyethyl Acrylate (MEA)

A NiTi base material (NiTi-PHA-MEA) in which MEA was polymerized via PHA was obtained in the same manner as in Example 1, except that, in "(4) Polymerization of N-Methacryloyloxyethyl-N,N-Dimethylammonium-α-N-Methylcarboxybetaine (CBA)", "2.5 g (19.21 mmol) of 2-methoxyethyl acrylate (MEA, 1.73 mol/L, manufactured by FUJIFILM Wako Pure Chemical Corporation)" was used instead of "1.97 g (monomer concentration: 20 mass%) of N-methacryloyloxyethyl-N,N-dimethylammonium-α-N-methylcarboxybetaine (CBA, manufactured by OSAKA ORGANIC CHEMICAL INDUSTRY LTD.).

### [Example 3]

### <Production of NiTi-PHA-MC3A>

### (4) Polymerization of 3-Methoxypropyl Acrylate (MC3A)

A NiTi base material (NiTi-PHA-MC3A) in which MC3A was polymerized via PHA was obtained in the same manner as in Example 1, except that, in "(4) Polymerization of N-Methacryloyloxyethyl-N,N-Dimethylammonium-α-N-Methylcarboxybetaine (CBA)", "2.48 g (17.20 mmol) of 3-methoxypropyl acrylate (MC3A, 1.73 mol/L, manufactured by TOKYO CHEMICAL INDUSTRY CO., LTD.)" was used instead of "1.97 g (monomer concentration: 20 mass%) of N-methacryloyloxyethyl-N,N-dimethylammonium-α-N-methylcarboxybetaine (CBA, manufactured by OSAKA ORGANIC CHEMICAL INDUSTRY LTD.).

### [Example 4]

### <Production of NiTi-PHA-MPC>

### (4) Polymerization of 2-(Methacryloyloxy)ethyl 2-(Trimethylammonio)ethyl Phosphate (another name: 2-methacryloyloxyethyl phosphorylcholine, MPC)

A NiTi base material (NiTi-PHA-MPC) in which MPC was polymerized via PHA was obtained in the same manner as in Example 1, except that, in "(4) Polymerization of N-Methacryloyloxyethyl-N,N-Dimethylammonium-α-N-Methylcarboxybetaine (CBA)", "1.477 g (5.00 mmol) of 2-(methacryloyloxy)ethyl 2-(trimethylammonio)ethyl phosphate (MPC, 0.5 mol/L, manufactured by TOKYO CHEMICAL INDUSTRY CO., LTD.)" was used instead of "1.97 g (monomer concentration: 20 mass%) of N-methacryloyloxyethyl-N,N-dimethylammonium-α-N-methylcarboxybetaine (CBA, manufactured by OSAKA ORGANIC CHEMICAL INDUSTRY LTD.).

### <Platelet Adhesion Test>

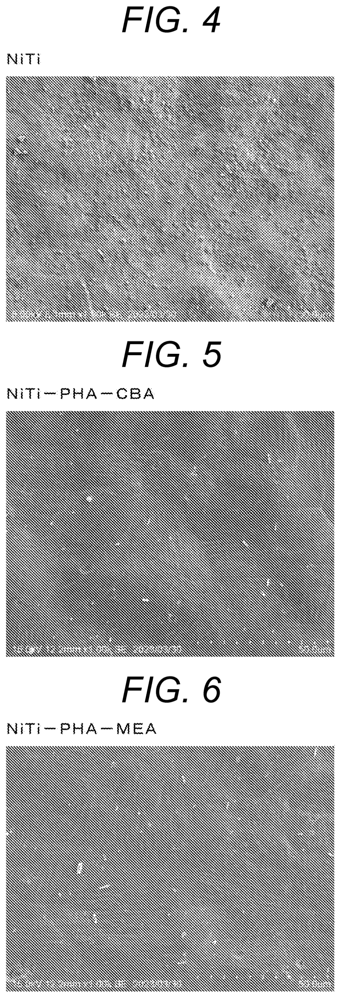

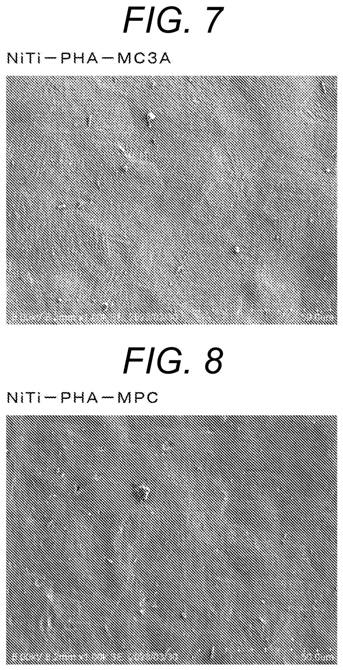

Using the samples and NiTi (not plasma-treated) produced as above, a platelet adhesion test was performed as follows, and antithrombogenicity was evaluated. A 3.2 (w/v)% sodium citrate (anticoagulant) aqueous solution was added to fresh porcine blood to obtain anticoagulated fresh porcine blood (1). In this case, the fresh porcine blood and the sodium citrate aqueous solution were mixed at a mixing ratio (volume ratio) of 9:1. A 1 M calcium chloride solution and heparin were simultaneously added to the anticoagulated fresh porcine blood (1) with a stirring bar to obtain anticoagulated fresh porcine blood (2). In this case, 820 µL of the 1 M calcium chloride solution and 1,000 U of the heparin were mixed and contained in 50 mL of the anticoagulated fresh porcine blood (2). The final heparin concentration in the anticoagulated fresh porcine blood (2) is 2 U/mL. 100 µL of the anticoagulated fresh porcine blood (2) was centrifuged (1500×g) at 22°C for 10 minutes to collect platelet-rich plasma (PRP). The PRP was filled into a cylindrical container in which each of the samples was attached to a bottom surface, and was left at room temperature overnight. Each sample was then cleaned with a 3.2 wt% sodium citrate/phosphate buffer solution (PBS) (=1/9 (volume ratio)) and fixed with 1 (w/v)% glutaraldehyde PBS solution. After leaving at room temperature for 4 hours, cleaning was performed with distilled water. Each sample was dried at room temperature, and platinum was deposited thereon. Then, the sample was observed and photographed (1,000x magnification, 5 visual fields) with a scanning electron microscope (SEM). The number of adhered platelets was counted for each photography and calculated in total for the five visual fields. The results are shown in Table 1 below. The SEM photographs of the examples are shown in Figs. 4 to 8. Fig. 4 is an SEM image of a NiTi base material (NiTi (not plasma-treated)) before a plasma treatment in Comparative Example 1. Fig. 5 is an SEM image of a NiTi base material (NiTi-PHA-CBA) of Example 1 in which CBA is polymerized via PHA. Fig. 6 is an SEM image of a NiTi base material (NiTi-PHA-MEA) of Example 2 in which MEA is polymerized via PHA. Fig. 7 is an SEM image of a NiTi base material (NiTi-PHA-MC3A) of Example 3 in which MC3A is polymerized via PHA. Fig. 8 is an SEM image of a NiTi base material (NiTi-PHA-MPC) of Example 4 in which MPC is polymerized via PHA.

### [Table 1]

**Table 1**

| | Number of Adhered Platelets | |
|---|---|---|
| NiTi (Not Plasma-Treated) | 1661 | Comparative Example 1 |
| NiTi-PHA-CBA | 214 | Example 1 |
| NiTi-PHA-MEA | 162 | Example 2 |
| NiTi-PHA-MC3A | 169 | Example 3 |
| NiTi-PHA-MPC | 150 | Example 4 |

As shown in Table 1 and Figs. 4 to 8, in the samples of Examples 1 to 4 according to the present invention, the adhesion (adhesion) of platelets to the surface of the NiTi base material was significantly reduced. From this, it is found that, according to the present invention, excellent functionality (antithrombogenicity) can be imparted to a medical device including a metal base material.

The present application is based on Japanese Patent Application No. 2023-098416 filed June 15, 2023, the entire content of which is incorporated herein by reference.

### Reference Signs List

- 10: Metal base material
- 10a: Base material core portion
- 10b: Base material surface layer
- 11: Antithrombotic layer
- 100: Medical device

## Claims

1. A medical device comprising:
a metal base material; and
a functional layer having a polymer including a constituent unit A derived from a bonding monomer having a phosphoric acid group or a phosphonic acid group and an ethylenically unsaturated group, which is bonded to the base material, and a functional polymer chain formed of a constituent unit B derived from a functional monomer, with a terminal of the functional polymer chain being bonded to the constituent unit A.

2. The medical device according to claim 1, wherein a ratio of a number of the constituent units B to a number of the constituent units A in the polymer is 30 or more.

3. The medical device according to claim 1, wherein the bonding monomer is one or more kinds selected from monomers represented by Formulae (I) and (II): 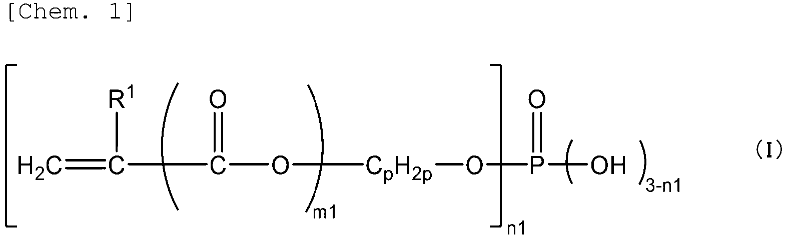
in Formula (I),
R¹ represents a hydrogen atom or a methyl group,
p represents an integer of 0 to 20,
m1 represents 0 or 1, and
n1 represents 1 or 2; and 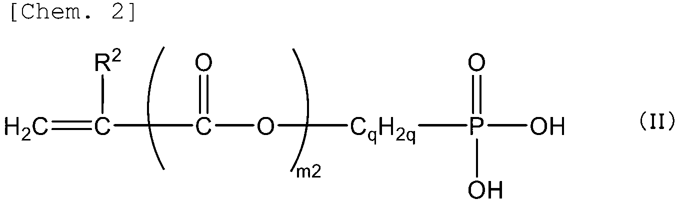
in Formula (II),
R² represents a hydrogen atom or a methyl group,
q represents an integer of 0 to 20, and
m2 represents 0 or 1.

4. The medical device according to claim 1, wherein the bonding monomer is one or more kinds selected from 2-(meth)acryloyloxyethyl acid phosphate, bis[2-((meth)acryloyloxy)ethyl] phosphate, 11-phosphonoundecyl (meth)acrylate, vinylphosphonic acid, and allylphosphonic acid.

5. The medical device according to claim 1, wherein the functional monomer is one or more kinds selected from monomers represented by Formulae (III) to (V): 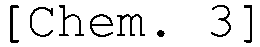 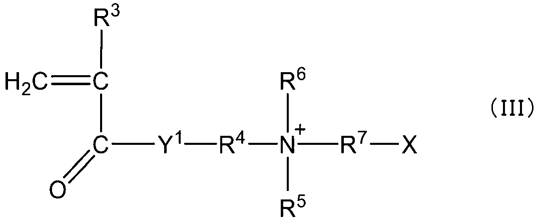
in Formula (III),
R³ represents a hydrogen atom or a methyl group,
R⁴ represents a linear or branched alkylene group having 1 to 6 carbon atoms,
R⁵ and R⁶ each independently represent a linear or branched alkyl group having 1 to 4 carbon atoms,
R⁷ represents a linear or branched alkylene group having 1 to 4 carbon atoms,
X represents -COO⁻ or -SO₃⁻, and
Y¹ represents an oxygen atom or -NH-; 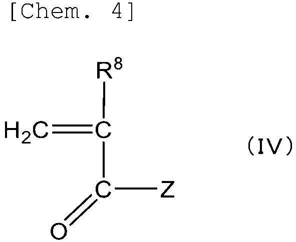
in Formula (IV),
R⁸ represents a hydrogen atom or a methyl group,
Z represents a group represented by Formula: -O-(R⁹O)ₘ-R¹⁰, a group represented by Formula: -O-(CH₂CHOH)ₙ-CH₂OH, or a tetrahydrofurfuryloxy group, in which R⁹ each independently represents a linear or branched alkylene group having 1 to 4 carbon atoms, R¹⁰ represents a linear or branched alkyl group having 1 to 4 carbon atoms, m represents an integer of 1 to 5, and n represents an integer of 1 to 5; and 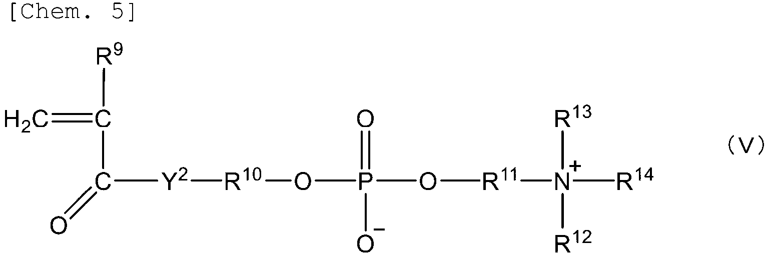
in Formula (V),
R⁹ represents a hydrogen atom or a methyl group,
R¹⁰ and R¹¹ each independently represent a linear or branched alkylene group having 1 to 6 carbon atoms,
R¹² to R¹⁴ each independently represent a linear or branched alkyl group having 1 to 4 carbon atoms, and
Y² represents an oxygen atom or -NH-.

6. The medical device according to claim 1, wherein the functional monomer is one or more kinds selected from N-(meth)acryloyloxymethyl-N,N-dimethylammonium-α-N-methylcarboxybetaine, N-(meth)acryloyloxyethyl-N,N-dimethylammonium-α-N-methylcarboxybetaine, N-(meth)acryloyloxypropyl-N,N-dimethylammonium-α-N-methylcarboxybetaine, N-(meth)acryloyloxymethyl-N,N-diethylammonium-α-N-methylcarboxybetaine, N-(meth)acryloyloxyethyl-N,N-diethylammonium-α-N-methylcarboxybetaine, N-(meth)acryloyloxypropyl-N,N-diethylammonium-α-N-methylcarboxybetaine, methoxymethyl acrylate, 2-methoxyethyl acrylate, 3-methoxypropyl acrylate, ethoxymethyl acrylate, ethoxyethyl acrylate, 2-(2-ethoxyethoxy)ethyl acrylate, ethoxypropyl acrylate, ethoxybutyl acrylate, propoxymethyl acrylate, butoxyethyl acrylate, methoxybutyl acrylate, tetrahydrofurfuryl acrylate, methoxymethyl methacrylate, methoxyethyl methacrylate, ethoxymethyl methacrylate, ethoxyethyl methacrylate, 2-(2-ethoxyethoxy)ethyl methacrylate, propoxymethyl methacrylate, butoxyethyl methacrylate, tetrahydrofurfuryl methacrylate, glycerol (meth)acrylate, and 2-methacryloyloxyethyl phosphorylcholine.

7. The medical device according to claim 1, wherein the metal is one or more kinds selected from the group consisting of gold, platinum, silver, copper, nickel, cobalt, titanium, iron, aluminum, tin, stainless steel, nickel-titanium alloys, nickel-cobalt alloys, cobaltchromium alloys, and zinc-tungsten alloys.

8. The medical device according to claim 1, wherein the medical device is a stent graft, a covered stent, a stent, a guide wire, or a cardiac pacemaker.

9. A method for manufacturing the medical device according to claim 1, the method comprising:
irradiating at least a part of a surface of the base material with plasma;
bringing the bonding monomer into contact with the base material after irradiation with the plasma; and
bringing the functional monomer into contact with the base material after contact with the bonding monomer to form the functional polymer chain on the surface of the base material via the constituent unit A derived from the bonding monomer.
